(19) Europäisches Patentamt
European Patent Office
Office européen des brevets

(11) **EP 0 802 922 B1**

(12) **EUROPÄISCHE PATENTSCHRIFT**

(45) Veröffentlichungstag und Bekanntmachung des Hinweises auf die Patenterteilung:
**26.09.2001 Patentblatt 2001/39**

(21) Anmeldenummer: **95909796.5**

(22) Anmeldetag: **02.03.1995**

(51) Int Cl.[7]: **C07K 5/02**, C07K 5/06

(86) Internationale Anmeldenummer:
**PCT/EP95/00760**

(87) Internationale Veröffentlichungsnummer:
**WO 95/23810 (08.09.1995 Gazette 1995/38)**

(54) **AMINOSÄUREDERIVATE, VERFAHREN ZU IHRER HERSTELLUNG UND DIESE ENTHALTENDE PHARMAZEUTISCHE ZUSAMMENSETZUNGEN**

NOVEL AMINO ACID DERIVATIVES, METHOD OF PRODUCING THEM AND PHARMACEUTICAL COMPOSITIONS CONTAINING THESE COMPOUNDS

DERIVES D'ACIDES AMINES, LEUR PROCEDE DE PREPARATION ET COMPOSITIONS PHARMACEUTIQUES LES CONTENANT

(84) Benannte Vertragsstaaten:
**AT BE CH DE DK ES FR GB GR IE IT LI LU MC NL PT SE**

(30) Priorität: **03.03.1994 DE 4406884**
**03.03.1994 DE 4406885**

(43) Veröffentlichungstag der Anmeldung:
**29.10.1997 Patentblatt 1997/44**

(73) Patentinhaber:
- **Boehringer Ingelheim Pharma KG**
**55218 Ingelheim am Rhein (DE)**
Benannte Vertragsstaaten:
**BE CH DE DK ES FR GR IT LI LU MC NL PT SE AT**
- **Boehringer Ingelheim International GmbH**
**55218 Ingelheim am Rhein (DE)**
Benannte Vertragsstaaten:
**GB IE**

(72) Erfinder:
- **ESSER, Franz**
**D-55218 Ingelheim/Rhein (DE)**
- **SCHNORRENBERG, Gerd**
**D-55435 Gau-Algesheim (DE)**
- **DOLLINGER, Horst**
**D-55218 Ingelheim/Rhein (DE)**
- **JUNG, Birgit**
**D-55270 Schwabenheim (DE)**
- **BUERGER, Erich**
**D-55411 Bingen/Rhein (DE)**
- **SPECK, Georg**
**D-55218 Ingelheim am Rhein (DE)**

(74) Vertreter: **Laudien, Dieter, Dr.**
**Boehringer Ingelheim GmbH,**
**Binger Strasse 173**
**55216 Ingelheim (DE)**

(56) Entgegenhaltungen:
**EP-A- 0 166 357** **WO-A-92/16524**
**WO-A-94/05693**

- **SCIENCE, Bd. 260, 1993 Seiten 1640-43, R.G.SMITH ET AL. 'A Nonpeptidyl Growth Hormone Secretagogue'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 802 922 B1

Printed by Jouve, 75001 PARIS (FR)

## Beschreibung

Die Erfindung betrifft neue Aminosäurederivate der allgemeinen Formel I

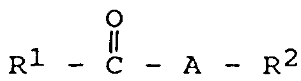

(I)

und deren pharmazeutisch annehmbare Salze, Verfahren zu ihrer Herstellung und diese Verbindungen enthaltende pharmazeutische Zusammensetzungen. Die Verbindungen sind wertvolle Neurokinin (Tachykinin)-Antagonisten.

In den europäischen Patentanmeldungen EP 394 989 und EP 443 132 werden Peptide mit Neurokinin antagonistischer Wirkung beschrieben.

In der nicht veröffentlichten deutschen Patentanmeldung DE 43 15 437 A1 und der korrespondierenden Anmeldung WO A 9405693 werden Verbindungen beschrieben, die sich von diesen Peptiden wesentlich in dem Glied $R^2$ unterscheiden.

Die neuen Verbindungen der vorliegenden Erfindung sind eine Auswahl unter der allgemeinen Definition der Verbindungen aus DE 43 15 437 A1 bzw. WO A 9405693.

Die in dieser Beschreibung und den Ansprüchen für die Aminosäuren verwendeten Abkürzungen entsprechen dem üblichen Dreibuchstabencode wie er.z.B. in Europ. J. Biochem., 138, 9 (1984) beschrieben ist. Die übrigen Abkürzungen werden nachfolgend erklärt:

| | |
|---|---|
| Boc = | t-Butoxycarbonyl |
| Bzl = | Benzyl |
| CDI = | Carbonyldiimidazol |
| DCCI = | Dicyclohexylcarbodiimid |
| DCH = | Dicyclohexylharnstoff |
| HOBt = | 1-Hydroxybenztriazol |
| Hyp = | (2S,4R)-Hydroxyprolin |
| THF = | Tetrahydrofuran |
| TEA = | Triethylamin |
| TFA = | Trifluoressigsäure |
| Z = | Benzyloxycarbonyl |
| Me = | Methyl |
| Ac = | Acetyl |
| Et = | Ethyl |
| DMF = | Dimethylformamid |
| DPPA = | Diphenylphosphorylazid |
| PPA = | Polyphosphorsäure |
| RT = | Raumtemperatur |
| TBTU = | Benzotriazolyl-tetramethyl-uroniumtetrafluoroborat |

Der Ausdruck Aminosäure umfaßt (falls im folgenden Text nicht ausdrücklich etwas anderes angegeben ist) natürliche und unnatürliche Aminosäuren, sowohl der D- als auch der L-Form, insbesondere α-Aminosäuren, sowie deren Isomere.

Wenn eine Aminosäure ohne Präfix angegeben ist (z.B. Orn) steht diese Angabe für die L-Form der Aminosäure. Die D-Form wird ausdrücklich angegeben.

Die Erfindung betrifft neue Aminosäurederivate der allgemeinen Formel I

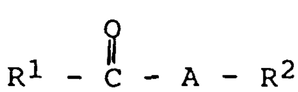

(I)

oder deren pharmazeutisch annehmbare Salze, worin

$R^1$ für Indolyl oder in Stellung 1 durch $C_1$-$C_3$ Alkyl oder Benzyl substituiertes Indolyl,

A D- oder L- Prolin (Pro), D- oder L-Didehydroprolin (ΔPro) wie beispielsweise 3,4-Didehydroprolin (Δ(3,4)-Pro), D- oder L-Hydroxyprolin (Pro(OH)) wie beispielsweise 3-Hydroxyprolin (Pro(30H)) und 4-Hydroxyprolin (Pro(40H)), D- oder L-Thioprolin (Tpr), D- oder L- Aminoprolin (Pro($NH_2$)) wie beispielsweise 3-Aminoprolin (Pro($3NH_2$)) und 4-Aminoprolin (Pro($4NH_2$)), D- oder L-Mercaptoprolin (Pro(SH)) wie beispielsweise 3-Mercaptoprolin (Pro(3SH)) und 4-Mercaptoprolin (Pro(4SH)), Tpr(O), oder Tpr($O_2$), bedeutet und deren geometrische Isomere, wobei enthaltene Hydroxy- und Aminogruppen durch übliche Schutzgruppen (z.B. Acyl, Carbamoyl oder Aralkyl (insbesondere Benzyl) geschützt sein können;

$R^2$ für ein Amin der Formel

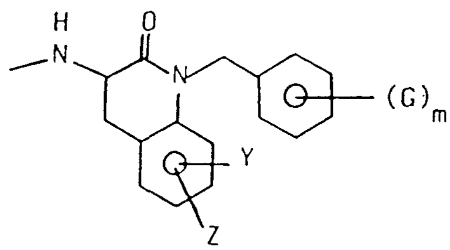

II

steht, worin

G Fluor, Chlor, Brom, Methyl, Ethyl oder Methoxy ist;

m 1, 2, 3, 4 oder 5 bedeutet;

Y und Z unabhängig voneinander für Wasserstoff, ($C_1$-$C_5$)-Alkyl, ($C_1$-$C_5$)-Alkyloxy, Benzyloxy [worin die Phenylgruppe unsubstituiert ist oder 1, 2 oder 3 Substituenten enthält, die unabhängig voneinander ($C_1$-$C_5$)-Alkyl, vorzugsweise Methyl, ($C_1$-$C_5$)- Alkoxy, vorzugsweise Methoxy, Dimethylamin, Halogen, Trifluormethyl, -CN oder $OCF_3$ sind], $OCF_3$, Halogen, $CF_3$, CN, $CH_2NH_2$, $CONH_2$, N-($C_1$-$C_5$-Alkyl)$_2$, NH-($C_1$-$C_4$)-alkylcarbonyl, N-($C_1$-$C_5$)-Alkyl-N-($C_1$-$C_4$)-alkylcarbonyl, $NH_2$ oder NH-($C_1$-$C_5$)-Alkyl stehen oder wenn Y und Z vicinal zueinander stehen, diese zusammen $-OCH_2O-$, $-OCH_2CH_2O-$ oder $-(CH_2)_4-$ bedeuten.

Verbindungen der allgemeinen Formel I können Säuregruppen besitzen, hauptsächlich phenolische Hydroxygruppen, und/oder basische Gruppen wie z.B. Guanidino- oder Aminofunktionen. Verbindungen der allgemeinen Formel I können deshalb entweder als innere Salze, als Salze mit pharmazeutisch verwendbaren anorganischen Säuren wie Salzsäure, Schwefelsäure, Phosphorsäure, Sulfonsäure oder organischen Säuren (wie beispielsweise Maleinsäure, Fumarsäure, Zitronensäure, Weinsäure oder Essigsäure) oder als Salze mit pharmazeutisch verwendbaren Basen wie Alkali- oder Erdalkalimetallhydroxiden oder Carbonaten, Zink- oder Ammoniumhydroxiden oder organischen Aminen wie z.B. Diethylamin, Triethylamin, Triethanolamin u.a. vorliegen.

Die Chiralitätszentren in den neuen Aminosäurederivaten können jeweils R-, S- oder R,S-Konfiguration besitzen.

Von den erfindungsgemäßen Verbindungen der Formel I sind solche bevorzugt worin

$R^1$ Indolyl oder in Stellung 1 durch Alkyl oder Benzyl substituiertes Indolyl bedeutet, und die Alkyl- gruppe 1 bis 3 Kohlenstoffatome enthält;

A D- oder L- Prolin (Pro), D- oder L-Didehydroprolin (ΔPro) wie beispielsweise 3,4-Didehydroprolin (Δ(3,4)-Pro), D- oder L-Hydroxyprolin (Pro(OH)) wie beispielsweise 3-Hydroxyprolin (Pro(30H)) und 4-Hydroxyprolin (Pro(40H)), D- oder

L-Thioprolin (Tpr), D- oder L- Aminoprolin (Pro($NH_2$)) wie beispielsweise 3-Aminoprolin (Pro($3NH_2$)) und 4-Aminoprolin (Pro($4NH_2$)), D- oder L-Mercaptoprolin (Pro(SH)) wie beispielsweise 3-Mercaptoprolin (Pro(3SH))

und 4-Mercaptoprolin (Pro(4SH)), Tpr(O), Tpr($O_2$) oder bedeutet und deren geometrische Isomere, wobei enthaltene Hydroxy- und Aminogruppen durch übliche Schutzgruppen (z.B. Acyl, Carbamoyl oder Aralkyl (insbesondere Benzyl) geschützt sein können, insbesondere solche, worin

$R^1$ Indolyl oder in Stellung 1 durch Alkyl oder Benzyl substituiertes Indolyl bedeutet; und die Alkyl- gruppe 1 bis 3 Kohlenstoffatome enthält, insbesondere

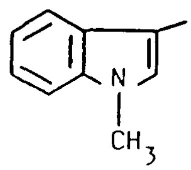

oder

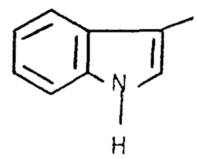

vorzugsweise, worin $R^1$

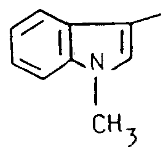

ist; und/oder worin

A eine Aminosäure ist, die keine oder eine polare funktionelle Gruppe in der Seitenkette trägt, wie OH, $NH_2$, SH; insbesondere

worin die funktionelle Gruppe in der Seitenkette von A OH ist, oder

worin A Pro oder 4-Hydroxyprolin ist, vorzugsweise

worin A 4-Hydroxyprolin mit 2-S-Konfiguration, insbesondere
ist.

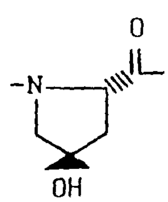

Von den erfindungsgemäßen Verbindungen der Formel I sind ferner solche bevorzugt, worin Y und Z unabhängig voneinander Methoxy oder Wasserstoff sind oder zusammen $-OCH_2O-$ bedeuten oder Y Wasserstoff und Z Clor ist.

Von den erfindungsgemäßen Verbindungen der Formel I sind ferner solche bevorzugt, worin

a. worin m 1 oder 2 ist,
b. worin G Chlor, Brom oder Ethyl ist, und
c. worin G Fluor ist und m 5.

Die angegebenen Aminosäuren liegen vorzugsweise in S-Konfiguration vor.

Die Verbindungen aus dem Disclaimer nach Anspruch 1 sind bereits aus dem Patent WO A 9405693 bekannt.

Untersuchungsergebnisse für erfindungsgemäße Verbindungen:

Die Rezeptoraffinität zum $NK_1$-Rezeptor (Substanz P-Rezeptor) wurde an humanen Lymphoblastoma-Zellen

(IM-9) mit klonierten $NK_1$-Rezeptoren bestimmt, wobei die Verdrängung von $^{125}J$-markierter Substanz P gemessen wird. Die so erhaltenen $IC_{50}$-Werte betragen:

| | |
|---|---|
| Verbindung Beispiel 1 | 0,4 nM |
| Verbindung Beispiel 6 | 0,75 nM |

Die erfindungsgemäßen Verbindungen sind wertvolle Neurokinin (Tachykinin)-Antagonisten, die insbesondere Substanz P-Antagonismus, aber auch Neurokinin A- bzw. Neurokinin-B-antagonistische Eigenschaften besitzen. Sie sind nützlich zur Behandlung von und zur Vorbeugung gegenüber Neurokinin-vermittelten Krankheiten wie Atemwegserkrankungen, z.B. Asthma, Bronchitits, Rhinitis, Husten oder Auswurf sowie von entzündlichen Augenkrankheiten wie beispielsweise Konjunktivits, von entzündlichen Hautkrankheiten wie beispielsweise Dermatitis und Urticaria, von anderen Entzündungskrankheiten wie Polyarthritis oder Osteoarthritis, von Magen-Darm-Krankheiten wie Reizcolon und Erbrechen, sowie von Schmerzzuständen wie z.B. Migräne.

Die Erfindung betrifft daher auch die Verwendung der erfindungsgemäßen Verbindungen als Heilmittel und pharmazeutische Zubereitungen, die diese Verbindungen enthalten. Bevorzugt ist die Anwendung am Menschen. Die Applikation der erfindungsgemäßen Verbindungen kann intravenös, subcutan, intramuskulär, intraperitoneal, intranasal, inhalativ, transdermal, gewünschtenfalls durch Iontophorese oder literaturbekannte Enhancer gefördert, und oral erfolgen.

Zur parenteralen Applikation werden die Verbindungen der Formel I oder deren physiologisch veträglichen Salze, eventuell mit den dafür üblichen Substanzen wie Lösungsvermittler, Emulgatoren oder weitere Hilfsstoffe in Lösung, Suspension oder Emulsion gebracht. Als Lösungsmittel kommen z.B. in Frage: Wasser, physiologische Kochsalzlösungen oder Alkohole, z.B. Ethanol, Propandiol oder Glycerin, Zuckerlösungen wie Glucose- oder Mannit-Lösungen oder auch eine Mischung aus verschiedenen Lösungsmitteln.

Außerdem können die Verbindungen durch Implantate, z.B. aus Polylactid, Polyglycolid oder Polyhydroxybuttersäure bzw. intranasale Zubereitungen appliziert werden.

Die erfindungsgemäßen Verbindungen können nach allgemein bekannten Methoden der Aminosäure- und Peptidchemie hergestellt werden.

Dabei werden die Komponenten $R^1$-COOH, die Aminosäure H-A-OH und das Amin H-$R^2$ miteinander verknüpft. Es kann wahlweise zuerst die Carbonsäure $R^1$-COOH mit einer geeignet geschützten Form von H-A-OH gekuppelt und nach Schutzgruppenabspaltung mit dem Amin H-$R^2$ kondensiert werden, oder es kann zuerst die geeignet geschützte Aminosäure H-A-OH mit H-$R^2$ umgesetzt und nach Deprotektion dieses Produkt mit $R^1$-COOH gekuppelt werden.

Die erfindungsgemäßen Grundkörper der Amine H-$R^2$ können nach an sich bekannten Verfahren gewonnen werden, z.B. nach A.L. Davis et al., J. Med. Chem. 18, 752 (1975) oder H. Merz, DE 38 23 576 (C.A. 114 (21), 207 052 m). Diese Herstellung kann durch das unten dargestellte Reaktionsschema zusammengefaßt werden. Die Einführung der Benzylgruppe R' in eine Verbindung der allgemeinen Formel XI erfolgt durch Umsetzen mit NaH und BrR', ClR' oder JR'. Diese Umsetzung kann ohne oder mit Verwendung einer Schutzgruppe (Sch) am exocyclischen N ausgeführt werden.

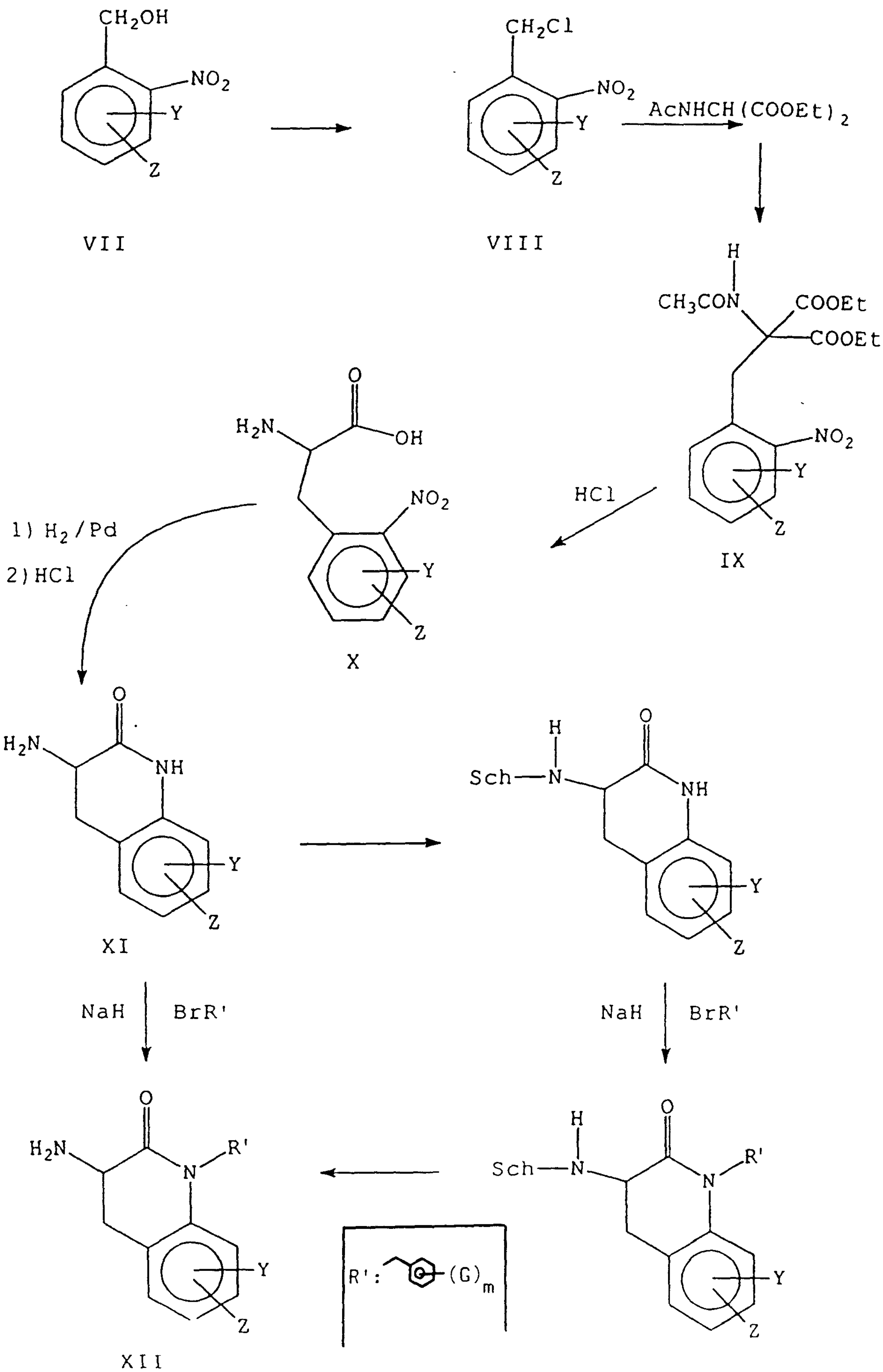


VII VIII IX X XI XII

Geeignete Schutzgruppen (Sch) sind basenstabile Schutzgruppen wie beispielsweise die Boc-gruppe.

Zur Herstellung einer Verbindung der allgemeinen Formel XI wird eine Verbindung der allgemeinen Formel X unter Ringschluß reduziert (z.B. analog zu der von A. L. Davis et al. (J. Med. Chem. 9, 826 (1966)) beschrieben mittels Pd-Mohr).

Die Verbindung X kann aus dem entsprechend substituierten 1-Nitrobenzylalkohol (VII) über die Zwischenstufen VIII und IX (durch Halogenieren mit z.B. $SOCl_2$ und anschließendem Umsetzen mit Acetamidomalonsäurediethylester nach J. Med. Chem. 9, 828 (1966)) hergestellt werden.

Zur Herstellung einer Verbindung der allgemeinen Formel XII kann an Stelle von BrR' auch allgemein HalR' eingesetzt werden, insbesondere ClR'.

Beispiel 1 und 2 (*R/S)

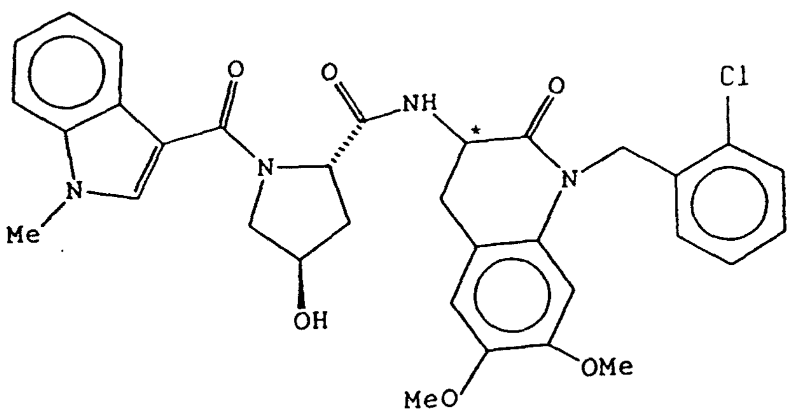

A. Herstellung der Ausgangsverbindung:

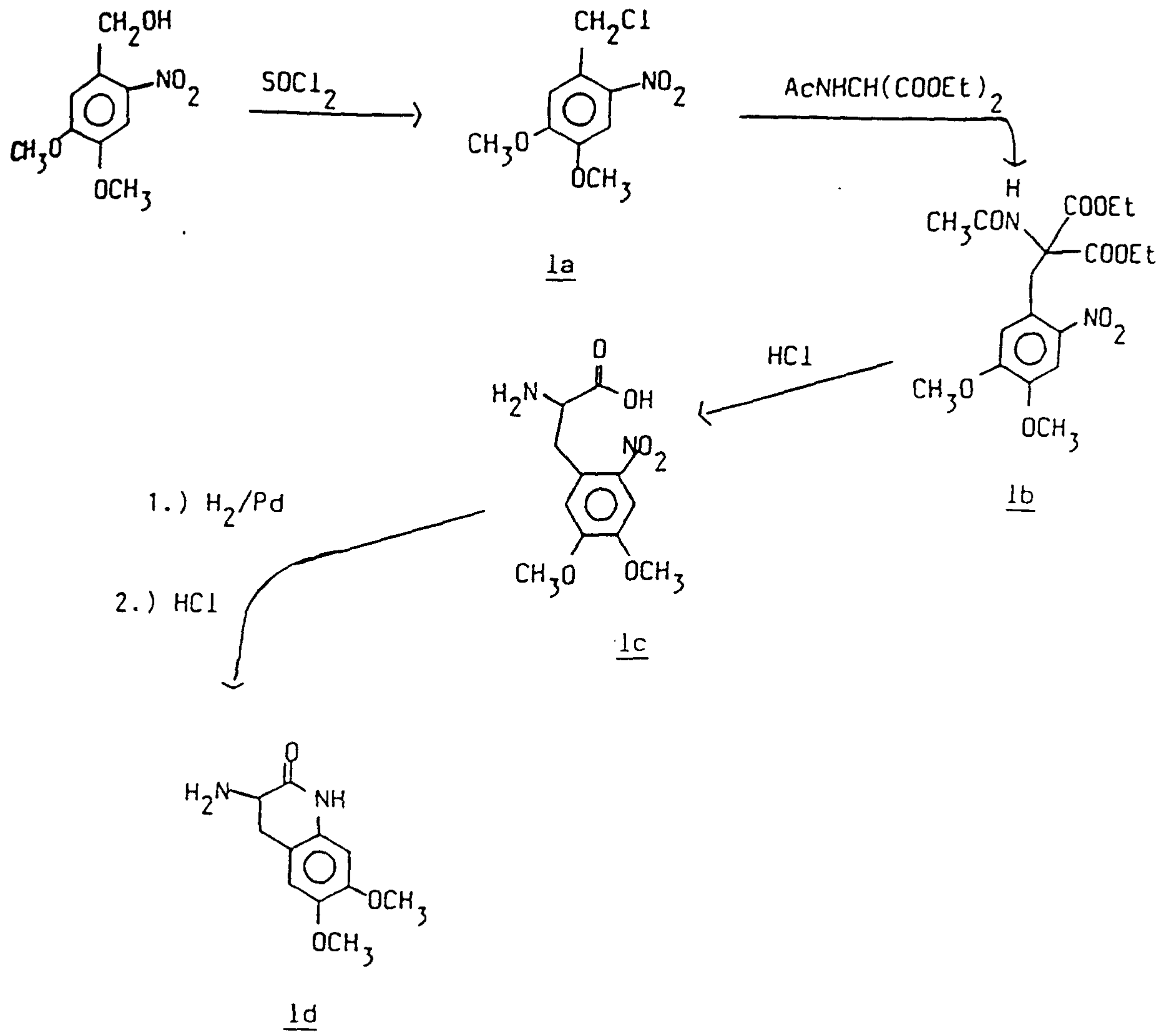


1a

1b

1c

1d

Herstellung von 1a:

10,7 g 6-Nitroveratrylalkohol wurden in 20 ml absolutem $SOCl_2$ und 2,7 ml absolutem Pyridin suspendiert, zum Sieden erhitzt und während 1/2h eine Mischung von 4 ml Thionylchlorid und 2 ml $CH_2Cl_2$ zugetropft. Es wurde noch 1h unter Rückfluß gekocht, dann abgekühlt und das Reaktionsgemisch in eine Mischung aus 20 g Eis und 20 g Wasser eingerührt. Die organische Phase wurde gründlich mit Wasser und $NaHCO_3$-Lösung gewaschen, mit $Na_2SO_4$ getrocknet und am Rotationsverdampfer eingeengt. Man erhielt 11,4 g 1a als dunkelbraunes Öl (Ausbeute 98%).

Herstellung von 1b:

11,2 g 1a wurden, wie in J. Med. Chem. 9, 828 (1966) beschrieben, mit 10,5 g Acetamidomalonsäurediethylester umgesetzt, wobei 16,2 g 1b in Form gelber Kristalle erhalten wurden (Ausbeute 81 %). Fp.: 176-178°C.

Herstellung von 1c:

In Anlehnung an die Vorschrift von A.L. Davis (J. Med. Chem. 9, 828, (1966)) wurden 16 g 1b mit 120 ml konzentierter Salzsäure hydrolysiert, wobei zunächst 1c.HCl erhalten wurde. Dieses wurde mittels Ammoniak in die freie Aminosäure 1c überführt, wovon 7,4 g in Form grünlicher Kristalle erhalten wurden (Ausbeute 71 %).Fp.: ca.

207°C (Zers.).

Herstellung von 1d:

5,4 g 1c wurden, wie von A.L. Davis et al. (J. Med. Chem. 9, 828 (1966)) beschrieben, mittels 0,6 g Pd-Mohr hydriert. Die erhaltene Aminoverbindung wurde zusammen mit 68 ml Ethanol und 12 ml konzentrierter Salzsäure 1/2h unter Rühren am Rückfluß gekocht. Nach dem Abkühlen wurde mit 26 ml Ether versetzt, abgesaugt und der Niederschlag mit eiskaltem Ethanol und Ether gewaschen und bei 80°C getrocknet. Man erhielt 3,3 g 1d.HCl in Form einer hellgrauen Festsubstanz (Ausbeute 63 %). Fp.: ca. 296°C (Zers.).

B. 12,9 g (±)-3-Amino-6,7-dimethoxy-1,2,3,4-tetrahydrochinolin-2-on wurde in einer Mischung aus 125 ml Dioxan und 125 ml Wasser gelöst, mit 12 g $(Boc)_2O$ versetzt und unter Rühren wurden 2,9 g Soda eingetragen. Nach einer Stunde Rühren bei Raumtemperatur wurde der ausgefallene Niederschlag abgesaugt, mit Wasser gewaschen und getrocknet; so wurden 12,3 g (±)-3-Boc-amino-6,7-dimethoxy-1,2,3,4-tetrahydrochinolin-2-on erhalten. Fp. 155-157°C.

1 g der erhaltenen Substanz wurde in 15 ml DMF gelöst und mit 0,13 g NaH-Dispersion (60 % in Öl) versetzt. Nach 0,5 h wurde eine Lösung aus 0,38 ml 2-Chlorbenzylchlorid, gelöst in 5 ml DMF, zugegeben und 5 Stunden bei Raumtemperatur gerührt. Dann wurden 200 ml eiskaltes Wasser zugesetzt und die erhaltenen Kristalle abgesaugt, mit Wasser gewaschen und getrocknet. Es wurden 1,28 g (±)-3-Boc-amino-1-(2-chlorbenzyl)-6,7-dimethoxy-1,2,3,4-tetrahydrochinolin-2-on erhalten.

Zur Abspaltung der Boc-Gruppe wurden 1,26 g der oben erhaltenen Verbindung mit 32,5 ml 4 n HCl in Dioxan und 2,3 ml Anisol versetzt und 1,5 Stunden bei Raumtemperatur gerührt. Die Reaktionsmischung wurde unter vermindertem Druck eingeengt, der Rückstand mit 150 ml Ether verrührt und die erhaltenen Kristalle abgesaugt, mit Ether gewaschen und getrocknet. Es wurden 0,96 g (±)-3-Amino-1-(2-chlorbenzyl)-6,7-dimethoxy-1,2,3,4-tetrahydrochinolin-2-on Hydrochlorid erhalten.

Kupplungsschritt:

0,96 g der erhaltenen Verbindung wurden mit 0,72 g (2S,4R)-N-(1-methylindol-3-ylcarbonyl)-4-hydroxyprolin, 40 ml DMF, 0,8 ml Triethylamin (= TEA) und 0,84 g Benzotriazolyl-tetramethyl-uroniumtetrafluoroborat (= TBTU) vereint, durch Zugabe von TEA der pH-Wert auf 8,5 bis 9 eingestellt und 1,5 Stunden bei Raumtemperatur gerührt. Die Reaktionsmischung wurde in 400 ml gesättigte $NaHCO_3$-Lösung eingerührt, der entstandene Niederschlag abgesaugt, mit Wasser, Essigester und Ether gewaschen und getrocknet. Es wurden 0,92 g der Verbindung Beispiel 2 als ca. 1: 1 R/S-Diastereomerengemisch erhalten.
Fp.: 166-168°C. $[\alpha]D^{20}$ (DMSO)= - 74,0°.

Diastereomerentrennung: 0,84 g des erhaltenen Diastereomerengemisches wurden mit 25 ml $CH_2Cl_2$ und 25 ml Wasser versetzt, mehrmals geschüttelt und im Ultraschallbad behandelt und die Suspension 4 Tage bei Raumtemperatur stehen gelassen. Es wurde filtriert, die $CH_2Cl_2$-Phase getrocknet und eingeengt, wobei 0,16 g der Verbindung Beispiel 1 als reines Diastereomeres erhalten wurden.
Fp.: 130-132°C
$[\alpha]D^{20}$ (DMSO) = + 12,5°

Zusammenfassung von Beispielen, die in analoger Weise hergestellt werden können:

Tabelle 1

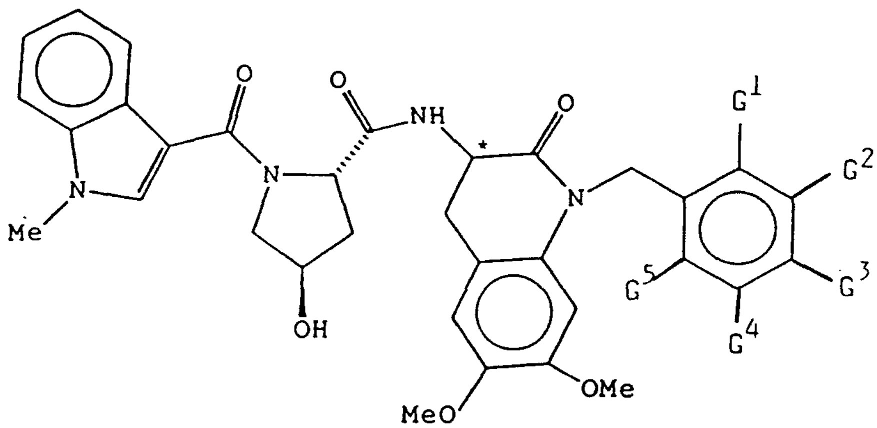

| Beispiel Nr. | $G^1$ | $G^2$ | $G^3$ | $G^4$ | $G^5$ | * |
|---|---|---|---|---|---|---|
| 1 | Cl | H | H | H | H | |
| 2 | Cl | H | H | H | H | R/S |
| 3 | Br | H | H | H | H | r.D. |
| 4 | Br | H | H | H | H | r.D. |
| 5 | F | H | H | H | H | R/S |
| 6 | $CH_2CH_3$ | H | H | H | H | r.D. |
| 7 | $CH_2CH_3$ | H | H | H | H | r.D. |
| 8 | Cl | Cl | H | H | H | |
| 9 | Cl | H | Cl | H | H | |
| 10 | F | F | F | F | F | |

r.D.: reines Diastereomeres

Beispiel 11

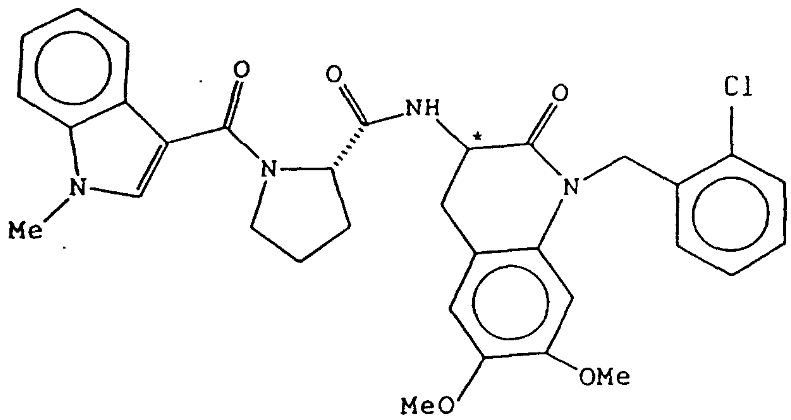

Beispiel 12

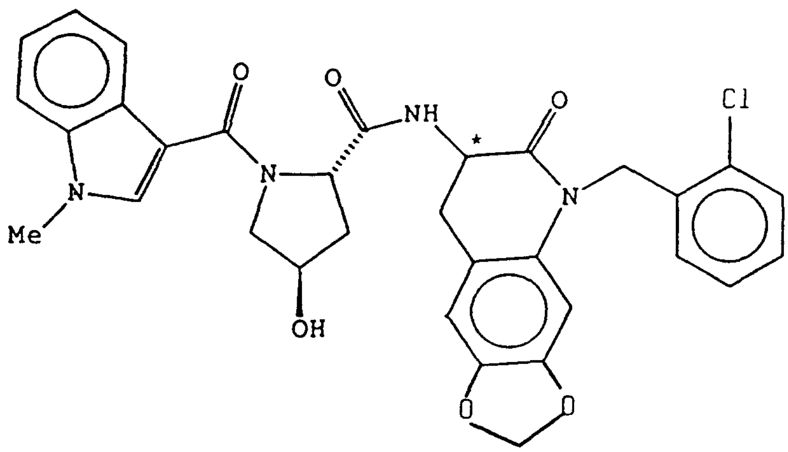

Physikalische Daten der Verbindungen:

Beispiel 3

Fp.: 100-110° (Zers.); $[\alpha]D^{20}$ (DMSO) = +13,8°

Beispiel 4

Fp.: 125-140° (Zers.); $[\alpha]D^{20}$ (DMSO) = -121,4°

Beispiel 5

Fp.: 141-146°C; $[\alpha]D^{20}$ (MeOH) = -71,0°

Beispiel 6

Fp.: 128-134°C (Zers.); $[\alpha]D^{20}$ (DMSO) = +0,6°

Beispiel 7

Fp.: 161-168°C; $[\alpha]D^{20}$ (DMSO) = -130,4°

Beispiel 8

($G^1$=$G^2$=Cl; $G^3$=$G^4$=$G^5$=H) wurde in die Diastereoisomeren getrennt.

Beispiel 8a

Fp.: 212-217°C; $[\alpha]_D^{20}$ (DMSO) = -143,6°

Beispiel 8b

Fp.: 148-152°C; $[\alpha]_D^{20}$ (DMSO) = +21,2°

Beispiel 10 ($G^1$-$G^5$= F; R/S;

Fp.: 143-153°C; $[\alpha]_D^{20}$ (DMSO) = -77,2°

Beispiel 13 und 14 (reine Diastereomere)

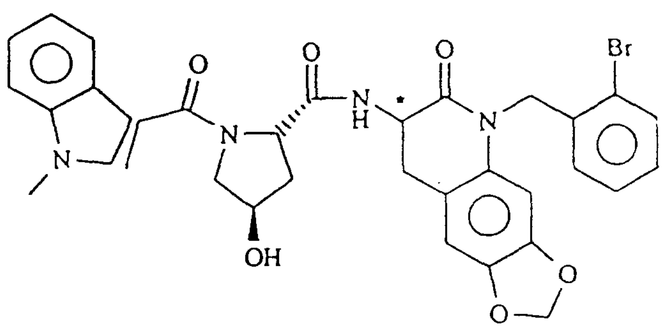

Beispiel 13

Fp.: 186-196°C; $[\alpha]_D^{20}$ (DMSO) = -119,8°

Beispiel 14

Fp.: ab 110°C Zers.; $[\alpha]_D^{20}$ (DMSO) = +3,6°

Beispiel 15

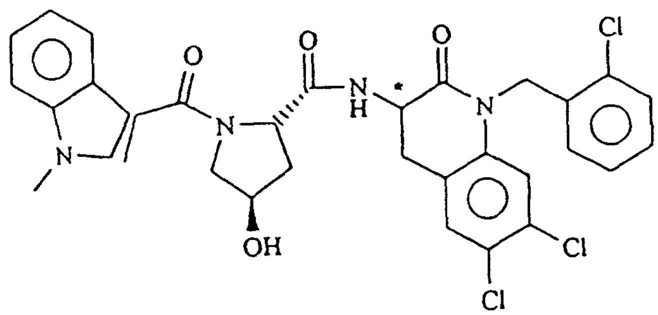

• R/S

Fp.: 244-255°C; $[\alpha]_D^{20}$ (DMSO) = -46,1°

Beispiel 16

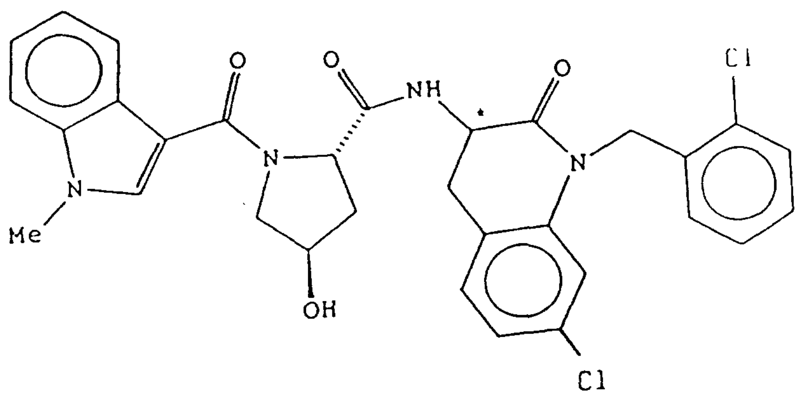

Als Ausgangsverbindung dient (±)-3-Amino-7-chlor-1,2,3,4-tetrahydrochinolin-2-on, dessen Herstellung in der Literatur beschrieben ist (T.J. McCord et al., J. Heterocycl. Chem. 9, 119 (1972); A.L. Davis et al., J. Med. Chem. 18, 752 (1975)).

11,1 g dieser Verbindung werden in einer Mischung aus 125 ml Dioxan und 125 ml Wasser gelöst, mit $(Boc)_2O$ versetzt und unter Rühren 2,9 g Soda eingetragen. Nach einer Stunde Rühren bei Raumtemperatur wird der ausgefallene Niederschlag abgesaugt, mit Wasser gewaschen und getrocknet. Somit erhält man (±)-3-Boc-amino-7-chlor-1,2,3,4-tetrahydrochinolin-2-on. 0,9 g der erhaltenen Verbindung wird in 15 ml DMF gelöst und mit 0,13 g NaH-Dispersion (60 % in Öl) versetzt. Nach 0,5 Stunden wird eine Lösung aus 0,38 ml 2-Chlorbenzylchlorid, gelöst in 5 ml DMF, zugegeben und 5 Stunden bei Raumtemperatur gerührt. Dann versetzt man mit 200 ml eiskaltem Wasser, saugt die erhaltenen Kristalle ab, wäscht mit Wasser und trocknet sie. Somit erhält man (±)-3-Boc-amino-1-(2-chlorbenzyl)-7-chlor-1,2,3,4-tetrahydrochinolin-2-on.

1,2 g der so erhaltenen Verbindung werden mit 32,5 ml 4 n HCl in Dioxan und 2,3 ml Anisol versetzt und 1,5 Stunden bei Raumtemperatur gerührt. Die Reaktionsmischung wird unter vermindertem Druck eingeengt, der Rückstand mit 150 ml Ether verrührt, die erhaltenen Kristalle abgesaugt, mit Ether gewaschen und getrocknet. somit erhält man (±)-3-Amino-1-(2-chlorbenzyl)-7-chlor-1,2,3,4-tetrahydrohinolin-2-on Hydrochlorid.

Kupplungsschritt:

0,88 g des erhaltenen Hydrochlorids werden mit 0,72 g (2S, 4R)-N-(1-Methylindol-3-yl-carbonyl)-4-hydroxyprolin, 40 ml DMF, 0,8 ml TEA und 0,84 g TBTU vereint, durch Zugabe von TEA der pH-Wert auf 8,5 bis 9 eingestellt

und 1,5 Stunden bei Raumtemperatur gerührt. Die Reaktionsmischung wird in 400 ml gesättigter $NaHCO_3$-Lösung eingerührt, der entstandene Niederschlag abgesaugt, mit Wasser, Essigester und Ether gewaschen und getrocknet. Somit wird Verbindung Beispiel 16 als ca. 1:1 R/S-Diastereomerengemisch erhalten.

Tabelle 2

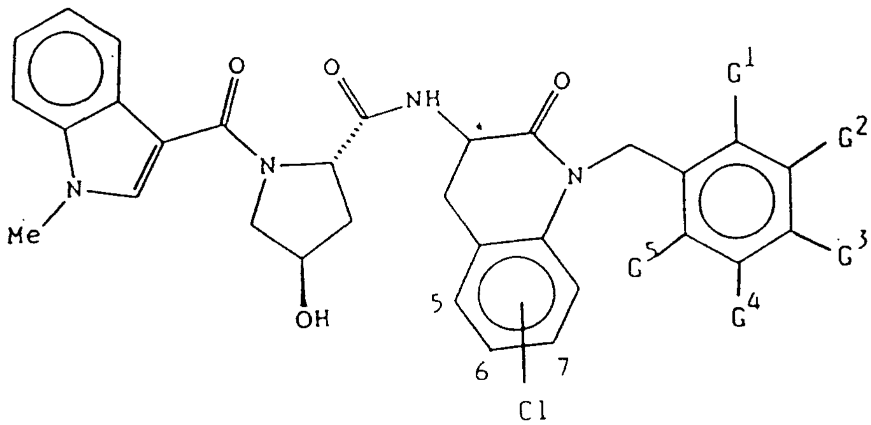

| Beispiel Nr. | $G^1$ | $G^2$ | $G^3$ | $G^4$ | $G^5$ | Cl in Position |
|---|---|---|---|---|---|---|
| 16 | Cl | H | H | H | H | 7 |
| 17 | Br | H | H | H | H | 7 |
| 18 | Cl | Cl | H | H | H | 7 |
| 19 | F | F | F | F | F | 7 |
| 20 | $CH_3$ | H | H | H | H | 7 |
| 21 | $CH_2CH_3$ | H | H | H | H | 7 |
| 22 | $OCH_3$ | H | H | H | H | 7 |
| 23 | Cl | H | H | H | H | 6 |
| 24 | Br | H | H | H | H | 6 |
| 25 | Cl | H | H | H | H | 5 |
| 26 | Br | H | H | H | H | 5 |
| 27 | Cl | H | H | H | H | 8 |

Beispiel 17

Fp.: 152-162°C; $[\alpha]D^{20}$ (MeOH)= -88,6°

Beispiel 23

Fp.: 158-195°C; $[\alpha]D^{20}$ (DMSO) = -48,2°

Beispiel 25

Fp.: 85-100°C; $[\alpha]D^{20}$ (DMSO) = -54,5°

Beispiel 27

Fp.: 78-100°C; $[\alpha]D^{20}$ (MeOH) = -85,2°

Beispiel 28

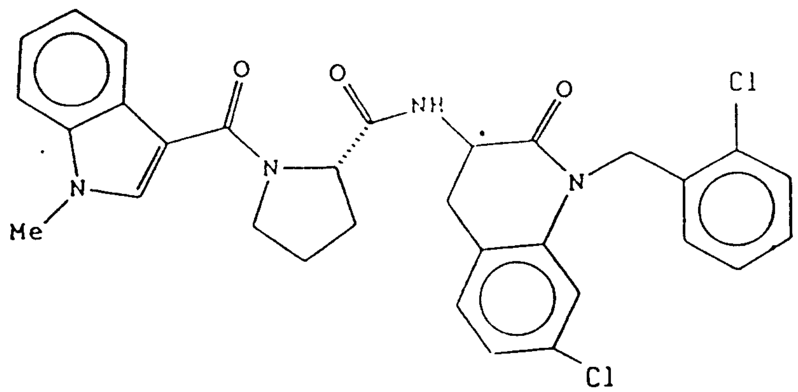

Pharmazeutische Zubereitungen:

| Injektionslösung | | |
|---|---|---|
| 200 mg | Wirksubstanz * | |
| 1,2 mg | Monokaliumdihydrogenphosphat = $KH_2PO_4$ | |
| 0,2 mg | Dinatriumhydrogenphosphat = (Puffer) | |
| | $NaH_2PO_4 \cdot 2H_2O$ | |
| 94 mg | Natriumchlorid | (Isotonans) |
| oder | | |
| 520 mg | Glucose | |
| 4 mg | Albumin | (Proteasenschutz) |
| q.s. | Natronlauge | |
| q.s. | Salzsäure | ad pH 6 |
| ad 10 ml | Wasser für Injektionszwecke | |
| Injektionslösung | | |
| 200 mg | Wirksubstanz* | |

* Wirksubstanz: erfindungsgemäße Verbindungen, z.B. die des Beispiels 1.

(fortgesetzt)

| | | |
|---|---|---|
| Injektionslösung | | |
| 94 mg<br>oder<br>520 mg | Natriumchlorid<br><br>Glucose | |
| 4 mg | Albumin | |
| q.s. | Natronlauge | |
| q.s. | Salzsäure | ad pH 9 |
| ad 10 ml | Wasser für Injektionszwecke | |
| Lyophilisat | | |
| 200 mg | Wirksubstanz* | |
| 520 mg | Mannit (Isotonans/Gerüstbildner) | |
| 4 mg | Albumin | |
| | | Lösungsmittel 1 für Lyophilisat |
| 10 ml | Wasser für Injektionszwecke | |
| | | Lösungsmittel 2 für Lyophilisat |
| 20 mg | Polysorbatò80 = Twenò80 (oberflächenaktiver Stoff) | |
| 10 ml | Wasser für Injektionszwecke | |

* Wirksubstanz: erfindungsgemäße Verbindungen, z.B. die des Beispiels 1.

Dosis für Mensch von 67 kg: 1 bis 500 mg

## Patentansprüche

**1.** Aminosäurederivat der allgemeinen Formel I

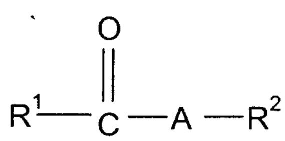

(I)

oder dessen pharmazeutisch annehmbares Salz, worin

- $R^1$ Indolyl, in Stellung 1 durch $C_1$-$C_3$ Alkyl oder Benzyl substituiertes Indolyl, bedeutet;
- A D- oder L- Prolin (Pro), D- oder L-Didehydroprolin (ΔPro), D- oder L-Hydroxyprolin (Pro(OH)), D- oder L-Thioprolin (Tpr), D- oder L- Aminoprolin (Pro($NH_2$)), D- oder L-Mercaptoprolin (Pro(SH)) bedeutet und deren geometrische Isomere, wobei enthaltene Hydroxy- und Aminogruppen durch Acyl, Carbamoyl oder Benzyl geschützt sein können;
- $R^2$ für ein Amin der Formel

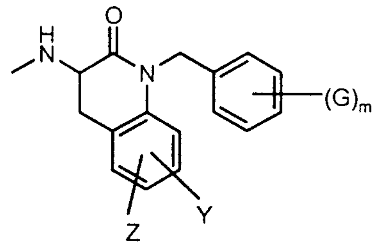

(II)

steht, worin

Y und Z unabhängig voneinander für Wasserstoff, ($C_1$-$C_5$)-Alkyl, ($C_1$-$C_5$)-Alkyloxy, Benzyloxy [worin die Phenylgruppe unsubstituiert ist oder 1, 2 oder 3 Substituenten enthält, die unabhängig voneinander ($C_1$-$C_5$)-Alkyl, ($C_1$-$C_5$)-Alkoxy, Dimethylamin, Halogen, Trifluormethyl, -CN oder $OCF_3$ sind], $OCF_3$, Halogen, $CF_3$, CN, $CH_2NH_2$, $CONH_2$, N-($C_1$-$C_5$-Alkyl)$_2$, NH-($C_1$-$C_4$)-Alkylcarbonyl, N-($C_1$-$C_5$)-Alkyl-N-($C_1$-$C_4$)-alkylcarbonyl, $NH_2$ oder NH-($C_1$-$C_5$)-Alkyl stehen oder wenn Y und Z vicinal zueinander stehen, diese zusammen $-OCH_2O-$,$-OCH_2CH_2O-$ oder $-(CH_2)_4-$ bedeuten;

G Fluor, Chlor, Brom, Methyl, Ethyl oder Methoxy und m 1, 2, 3, 4 oder 5 ist;

mit der Maßgabe, daß
Formel I nicht Formel III bis X

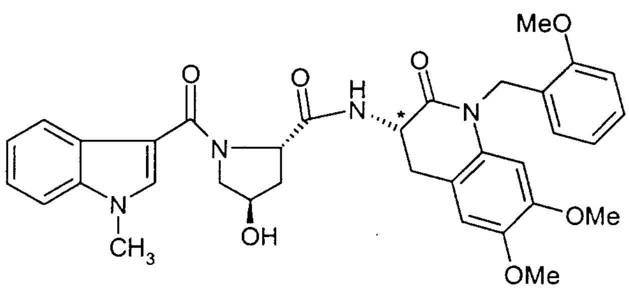

*S

(III)

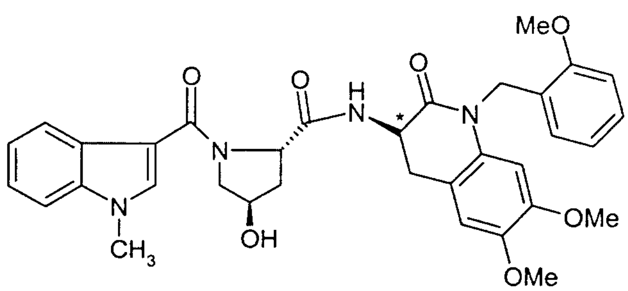

*R

(IV)

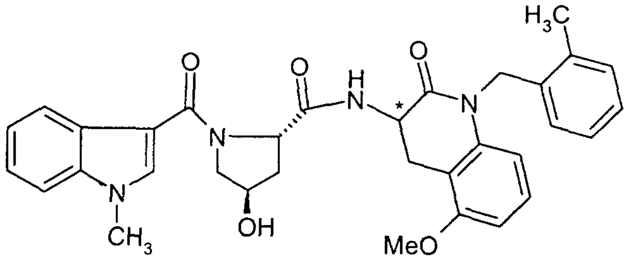

*R/S

(V)

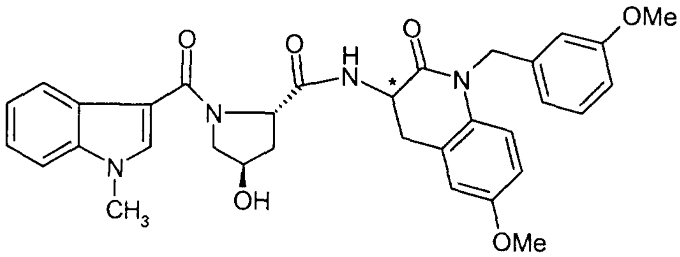

*R/S

(VI)

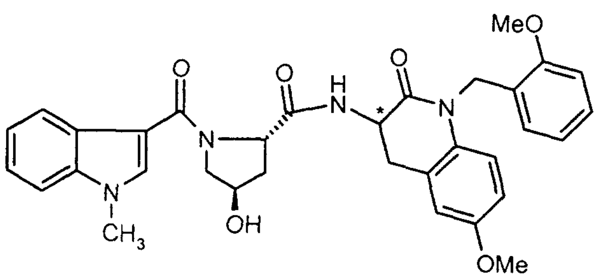

*R/S

(VII)

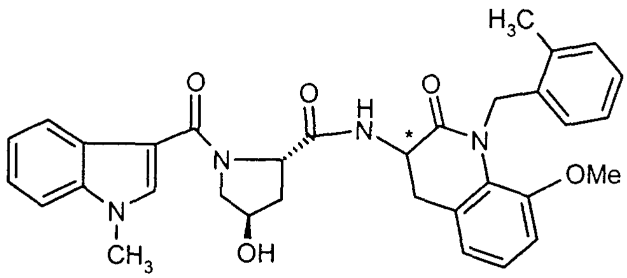

*R/S

(VIII)

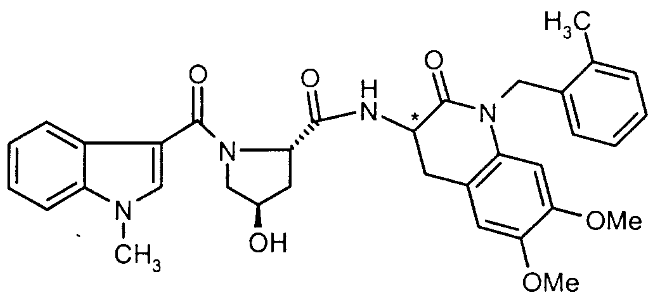

*S

(IX)

oder

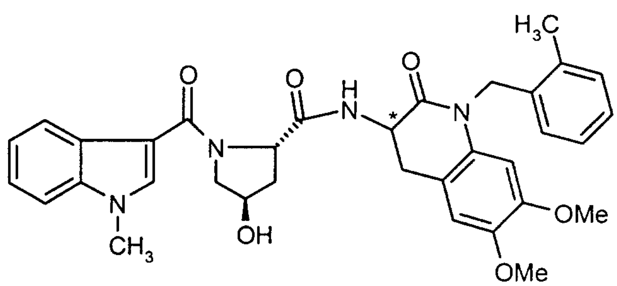

*R

(X)

sein kann.

**2.** Verbindung nach Anspruch 1, worin

$R^1$

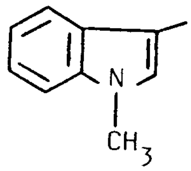

oder

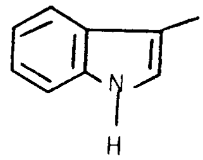

bedeutet.

**3.** Verbindung nach Anspruch 2, worin $R^1$

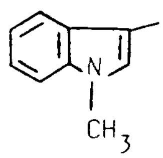

ist.

**4.** Verbindung nach einem der Ansprüche 1 bis 3, worin A Pro oder 4-Hydroxyprolin ist.

**5.** Verbindung nach Anspruch 4, worin A 4-Hydroxyprolin mit 2-S-Konfiguration, insbesondere

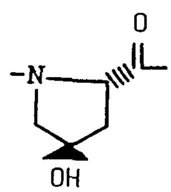

ist.

**6.** Verbindung nach einem der Ansprüche 1 bis 5, worin Y und Z unabhängig voneinander Methoxy oder Wasserstoff sind oder zusammen $-OCH_20-$ bedeuten oder Y Wasserstoff und Z Chlor ist.

**7.** Verbindung nach einem der Ansprüche 1-5, worin m 1 oder 2 ist.

**8.** Verbindung nach einem der Ansprüche 1-7, worin G Chlor, Brom oder Ethyl ist.

**9.** Verbindung nach einem der Ansprüche 1-5, worin G Fluor ist und m 5.

**10.** Verfahren zur Herstellung einer Verbindung nach einem der Ansprüche 1 bis 9 oder deren Salze, **dadurch gekennzeichnet, daß** man nach bekannten Methoden schrittweise die jeweiligen Aminosäuren, Carbonsäuren und Amine kondensiert und die so erhaltene Verbindung in freier Form oder in Form des gewünschten Salzes isoliert.

**11.** Pharmazeutische Zubereitung enthaltend eine Verbindung nach einem der Ansprüche 1 bis 9.

**12.** Verwendung einer Verbindung nach einem der Ansprüche 1 bis 9 zur Herstellung eines Medikamentes zur Therapie von und zur Vorbeugung gegenüber Neurokinin-vermittelten Krankheiten.

## Claims

**1.** Amino acid derivative of general formula I

$$R^1 - \overset{\overset{\displaystyle O}{\|}}{C} - A - R^2 \qquad (I)$$

or a pharmaceutically acceptable salt thereof, wherein

$R^1$ denotes indolyl, indolyl substituted in position 1 by $C_{1-3}$-alkyl or benzyl;

A denotes D- or L-proline (Pro), D- or L-didehydroproline (ΔPro), D- or L-hydroxyproline (Pro(OH)), D- or L-thioproline (Tpr), D- or L-aminoproline (Pro($NH_2$)), D- or L-mercaptoproline (Pro(SH)) and the geometric isomers thereof, whilst any hydroxy and amino groups which they may contain may be protected by acyl, carbamoyl or benzyl;

$R^2$ denotes an amine of formula II

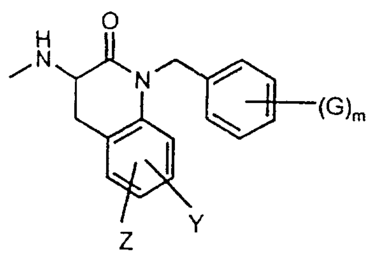

(II)

wherein

Y and Z independently of each other represent hydrogen, ($C_{1-5}$)alkyl, ($C_{1-5}$)alkyloxy, benzyloxy [wherein the phenyl group is unsubstituted or contains 1, 2 or 3 substituents which, independently of each other, are ($C_{1-5}$)alkyl, ($C_{1-5}$)alkoxy, dimethylamine, halogen, trifluoromethyl, -CN or $-OCF_3$], $-OCF_3$, halogen, $-CF_3$, -CN, $-CH_2NH_2$, $-CONH_2$, -N-($C_{1-5}$-alkyl)$_2$, -NH-($C_{1-4}$)-alkylcarbonyl, -N- ($C_{1-5}$) -alkyl-N- ($C_{1-4}$) -alkylcarbonyl, $NH_2$ or NH-($C_{1-5}$)-alkyl or, if Y and Z are arranged vicinally to each other, they together represent $-OCH_2O-$, $-OCH_2CH_2O-$ or $-(CH_2)_4-$;

G is fluorine, chlorine, bromine, methyl, ethyl or methoxy and

m denotes 1, 2, 3, 4 or 5;

with the proviso that formula I cannot be formulae III to X

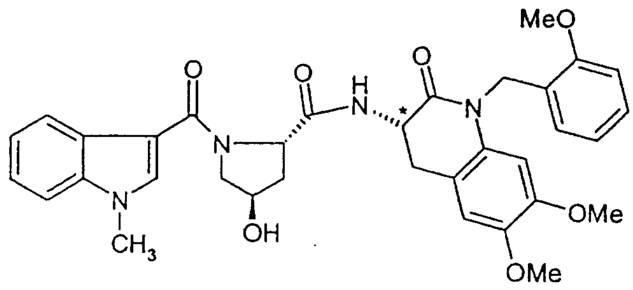

*S

(III)

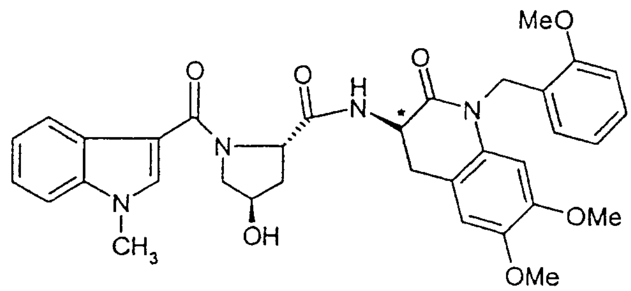

*R

(IV)

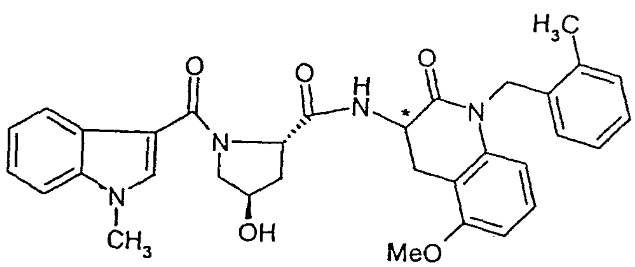

*R/S

(V)

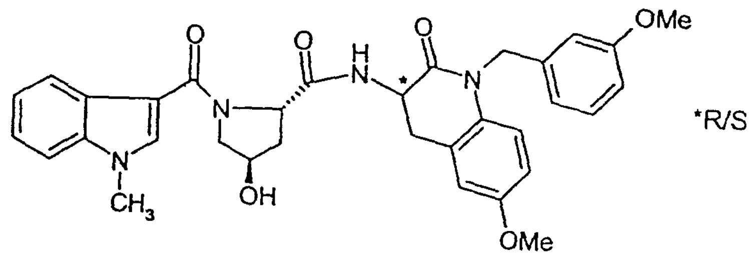

*R/S

(VI)

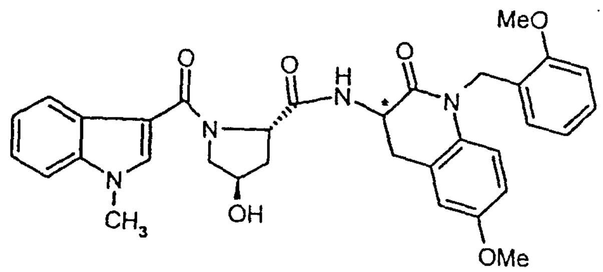

*R/S

(VII)

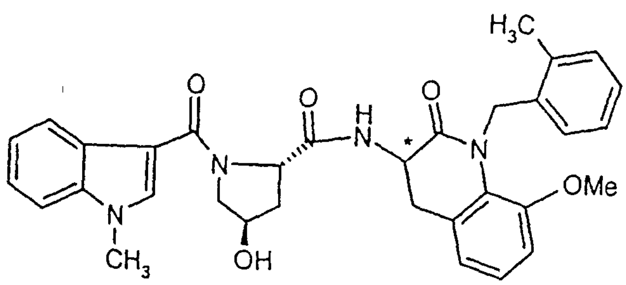

*R/S

(VIII)

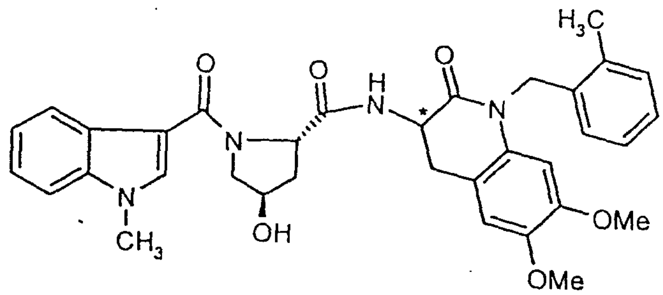

*S

(IX)

or

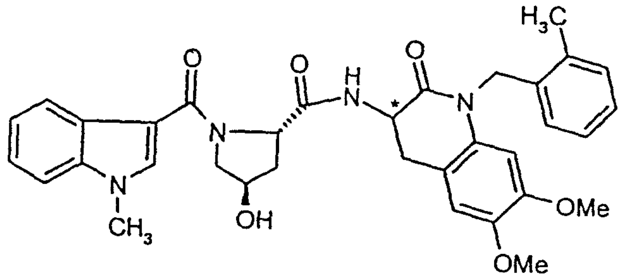

*R

(X)

2. Compound according to claim 1, wherein

R¹ denotes

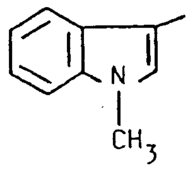

or

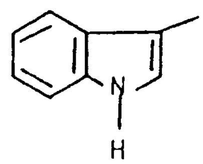

3. Compound according to claim 2, wherein

R¹ is

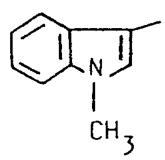

**4.** Compound according to one of claims 1 to 3, wherein A is Pro or 4-hydroxyproline.

**5.** Compound according to claim 4, wherein A is 4-hydroxyproline with the 2-S-configuration, particularly

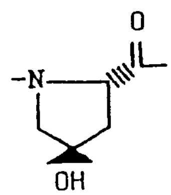

**6.** Compound according to one of claims 1 to 5, wherein Y and Z independently of each another denote methoxy or hydrogen or together represent $-OCH_2O-$ or Y is hydrogen and Z is chlorine.

**7.** Compound according to any one of claims 1 to 5, wherein m is 1 or 2.

**8.** Compound according to any one of claims 1 to 7, wherein G is chlorine, bromine or ethyl.

**9.** Compound according to any one of claims 1 to 5, wherein G is fluorine and m is 5.

**10.** Process for preparing a compound according to any one of claims 1 to 9 or the salts thereof, **characterised in that** the appropriate amino acids, carboxylic acids and amines are condensed step by step using known methods and the compound thus obtained is isolated in free form or in the form of the desired salt.

**11.** Pharmaceutical preparation containing a compound according to any one of claims 1 to 9.

**12.** Use of a compound according to one of claims 1 to 9 for the preparation of a medicament for the treatment and prevention of neurokinin-mediated diseases.

**Revendications**

**1.** Dérivé d'acide aminé de formule générale I

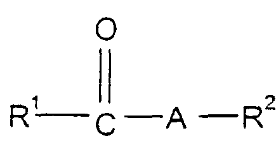

(I)

ou son sel pharmaceutiquement acceptable,
où

$R^1$ représente indolyle, indolyle substitué en position 1 par alkyle en $C_1$-$C_3$ ou benzyle ;
A représente la D- ou L-proline (Pro), la D- ou L-didéshydroproline (Δpro), la D- ou L-hydroxyproline (Pro(OH)), la D- ou L-thioproline (Tpr), la D- ou L-aminoproline (Pro($NH_2$)), la D- ou L-mercaptoproline (Pro(SH)) et leurs isomères géométriques, où les groupes hydroxyle et amino contenus peuvent être protégés par acyle, carbamoyle ou benzyle ;
$R^2$ représente une amine de formule

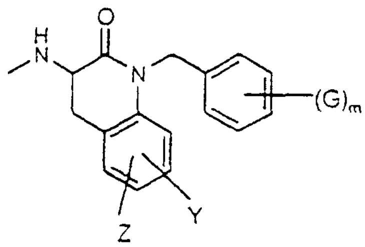

(II)

où

Y et Z représentent indépendamment l'un de l'autre l'hydrogène, alkyle en $C_1$-$C_5$, alkyloxy en $C_1$-$C_5$, benzyloxy [où le groupe phényle est non substitué ou contient 1, 2 ou 3 substituants qui sont, indépendamment les uns des autres, alkyle en $C_1$-$C_5$, alcoxy en $C_1$-$C_5$, diméthylamine, halogène, trifluorométhyle, -CN ou $OCF_3$], $OCF_3$, halogène, $CF_3$, CN, $CH_2NH_2$, $CONH_2$, N-(alkyle en $C_1$-$C_5$)$_2$, NH-alkyle en $C_1$-$C_4$-carbonyle, N-alkyle en $C_1$-$C_5$-N-alkyle en $C_1$-$C_4$-carbonyle, $NH_2$ ou NH-alkyle en $C_1$-$C_5$ ou, quand Y et Z sont voisins l'un de l'autre, ils représentent ensemble $-OCH_2O-$, $-OCH_2CH_2O-$ ou $-(CH_2)_4-$ ;

G est le fluor, le chlore, le brome, méthyle, éthyle ou méthoxy et

m est 1, 2, 3, 4 ou 5 ;

à condition que

la formule I ne puisse pas être les formules III à X

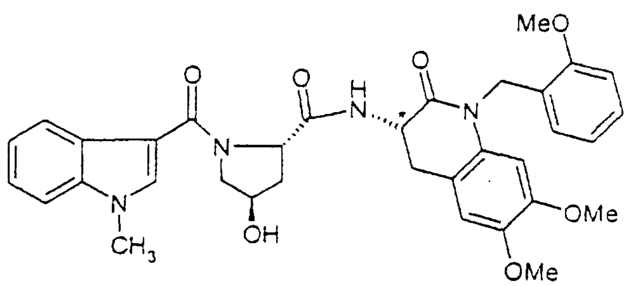

*S

(III)

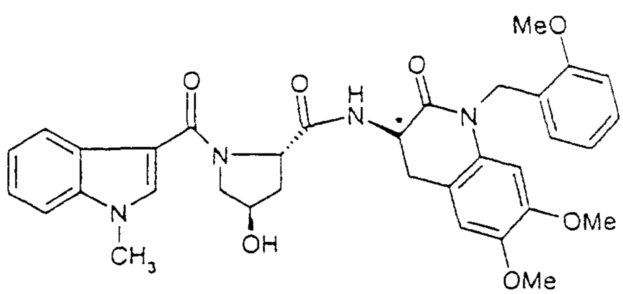

*R

(IV)

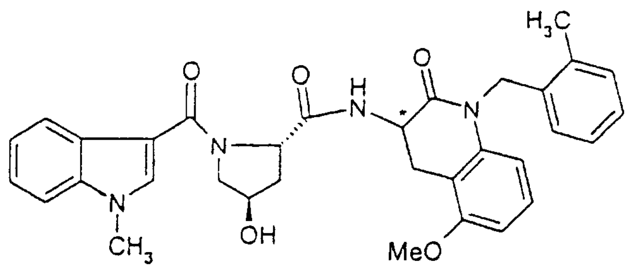

*R/S

(V)

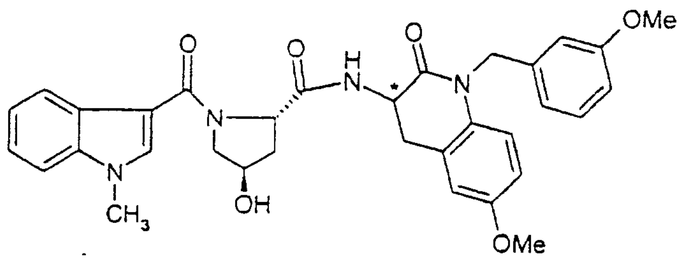

*R/S

(VI)

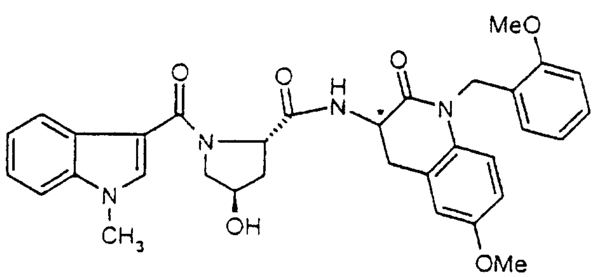

*R/S

(VII)

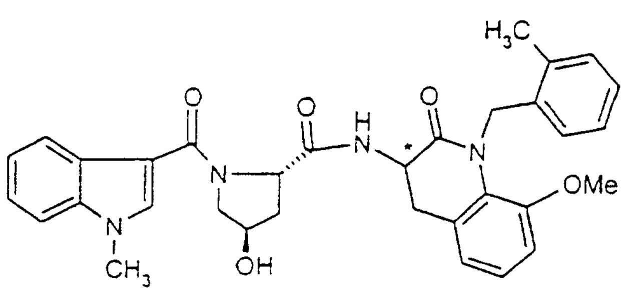

*R/S

(VIII)

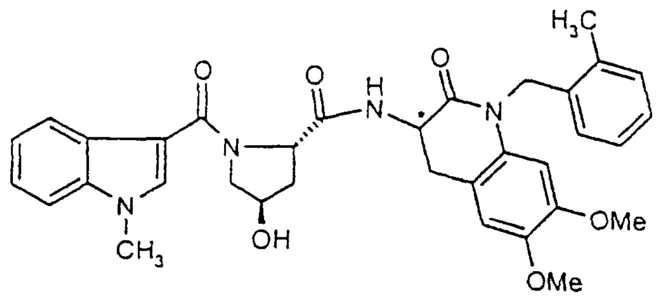

*S

(IX)

ou

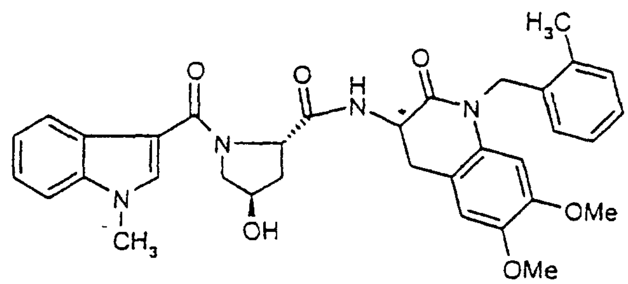

*R

(X)

2. Composé selon la revendication 1 où $R^1$ représente

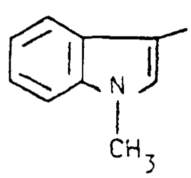

ou

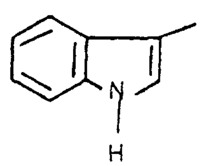

3. Composé selon la revendication 2 où $R^1$ est

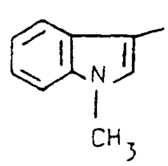

4. Composé selon l'une des revendications 1 à 3 où A est Pro ou la 4-hydroxyproline.

5. Composé selon la revendication 4 où A est la 4-hydroxyproline à configuration 2-S, en particulier

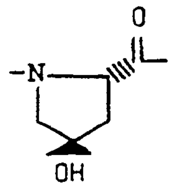

6. Composé selon l'une des revendications 1 à 5 où Y et Z sont indépendamment l'un de l'autre méthoxy ou l'hydrogène ou représentent ensemble -$OCH_2O$- ou bien Y est l'hydrogène et Z est le chlore.

7. Composé selon l'une des revendications 1-5 où m est 1 ou 2.

8. Composé selon l'une des revendications 1-7 où G est le chlore, le brome ou éthyle.

9. Composé selon l'une des revendications 1-5 où G est le fluor et m est 5.

10. Procédé de préparation d'un composé selon l'une des revendications 1 à 9 ou de ses sels **caractérisé en ce que**, selon des méthodes connues, par étapes, on condense les différents acides aminés, acides carboxyliques et amines et on isole le composé ainsi obtenu sous forme libre ou sous forme du sel voulu.

11. Préparation pharmaceutique contenant un composé selon l'une des revendications 1 à 9.

12. Utilisation d'un composé selon l'une des revendications 1 à 9 pour la préparation d'un médicament pour la thérapie et la prévention des maladies à médiation par des neurokinines.